(19) Europäisches Patentamt
European Patent Office
Office européen des brevets

(11) **EP 0 726 092 B1**

(12) **EUROPÄISCHE PATENTSCHRIFT**

(45) Veröffentlichungstag und Bekanntmachung des Hinweises auf die Patenterteilung:
**11.08.1999 Patentblatt 1999/32**

(51) Int. Cl.$^6$: **B01J 23/22**, B01J 23/18, C07D 213/84

(21) Anmeldenummer: **96100676.4**

(22) Anmeldetag: **18.01.1996**

(54) **Verfahren zur Herstellung von Cyanopyridinen und dafür geeignete Katalysatoren**

Cyanopyridine preparation process and catalysts therefor

Procédé de préparation de cyanopyridines et catalyseurs à cet usage

(84) Benannte Vertragsstaaten:
**BE CH DE FR GB LI NL**

(30) Priorität: **09.02.1995 DE 19504283**

(43) Veröffentlichungstag der Anmeldung:
**14.08.1996 Patentblatt 1996/33**

(73) Patentinhaber:
**Degussa-Hüls Aktiengesellschaft**
**45764 Marl (DE)**

(72) Erfinder:
 • **v. Hippel, Lukas, Dr.**
 **D-63755 Alzenau (DE)**
 • **Neher, Armin, Dr.**
 **D-50289 Wesseling (DE)**
 • **Arntz, Dietrich, Dr.**
 **D-61440 Oberursel (DE)**

(56) Entgegenhaltungen:
 EP-A- 0 055 534    EP-A- 0 059 414
 WO-A-95/05895     US-A- 4 508 848
 US-A- 4 814 478

 • **DATABASE WPI Section Ch, Week 8840 Derwent Publications Ltd., London, GB; Class B03, AN 88-283060 XP002003706 & JP-A-63 208 575 (NIPPON SHOKUBAI KAGAKU) , 30.August 1988**

Bemerkungen:
 Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Herstellung von Cyanopyridinen durch die katalytische Umsetzung von Methylpyridinen mit Ammoniak und Sauerstoff bei erhöhter Temperatur. Sie betrifft insbesondere die für diesen Zweck geeigneten Katalysatoren, die aus Verbindungen der Elemente Antimon, Vanadin, Silizium, Titan und Sauerstoff und Verbindungen eines oder mehrerer der Alkalimetalle bestehen, sowie das Verfahren zur Herstellung der Katalysatoren und ihre Verwendung.

Es sind mehrere Verfahren zur Herstellung von Cyanopyridinen durch Umsetzung der entsprechenden Methylpyridine mit Sauerstoff und Ammoniak bei erhöhter Temperatur in der Gasphase bekannt. Sie unterscheiden sich durch die Umsetzungsbedingungen und insbesondere durch die Katalysatorzusammensetzung. Unter den Verfahren und Katalysatoren sind nur solche für eine Anwendung im technischen Maßstab von Bedeutung, die eine gute Selektivität und Standzeit aufweisen und gleichzeitig eine hohe Raum-Zeit-Ausbeute ergeben.

Es ist weiter bekannt, Katalysatoren zu verwenden, die dadurch hergestellt werden, daß Mischungen, die Antimon und Vanadium im atomaren Verhältnis von 1,1 zu 1 bis 50 zu 1 und wenigstens eines der Elemente Eisen, Kupfer, Titan, Kobalt, Mangan und Nickel und gegebenenfalls eine Trägersubstanz enthalten, durch Erhitzen auf Temperaturen von 600 bis 1100°C in Gegenwart von Sauerstoff vorbehandelt werden (DE 20 39 497). Bei diesem Verfahren werden zwar hohe Raum-Zeit-Ausbeuten erzielt, die Selektivität ist jedoch unbefriedigend.

Es ist bekannt, daß Katalysatoren zur Ammonoxidation von Methylpyridinen auf der Basis von Titanoxid-Siliciumoxid-Trägern hergestellt werden können, die mit den Oxiden des Antimons und des Vanadiums bedeckt sind (EP 0 290 996 B1 und US 4,939,260). Diese Katalysatoren zeigen im Fall der Ammonoxidation von 3-Methylpyridin nur Ausbeuten um 85 %.

Es ist ferner bekannt, daß Katalysatoren zur Herstellung von 3-Cyanopyridin mit guter Selektivität und hoher Raum-Zeit-Ausbeute aus Schichtgittersilikaten, hochdispersem Siliciumoxid und Sauerstoffverbindungen der Elemente Antimon und Vanadium und mindestens einem der Elemente Eisen, Kupfer, Titan, Kobalt` Mangan und Nickel hergestellt werden können (EP 0 059 414 B1). Die Herstellung dieser Katalysatoren ist sehr aufwendig und fordert unter anderem eine Zwischenkalzinierung mit nachfolgender Mahlung.

In der JP-A 63/208575 wird ein Verfahren zur Herstellung von Cyanopyridin beschrieben, bei dem man einen Trägerkatalysator, insbesondere auf Basis Anatas oder Siliciumcarbid einsetzt.

Dieser erweist sich jedoch als sehr empfindlich gegen eine nicht optimale Abteilung der Reaktionswärme.

Aufgabe der vorliegenden Erfindung war es, Katalysatoren zu finden, deren Herstellung vergleichsweise einfach ist und die bei hohen Umsätzen hohe Selektivitäten in Kombination mit hohen Raum-Zeit-Ausbeuten erreichen.

Es sind nun Katalysatoren für die Umsetzung von Methylpyridinen mit Ammoniak und Sauerstoff zu den entsprechenden Cyanopyridinen gefunden worden, die der allgemeinen Summenformel

$$Sb_a V_b Ti_c X_d^1 X_e^2 O_f$$

entsprechen, in der bedeuten

$X^1$    Silicium, wobei das Si aus dem bei der Herstellung eingebrachten hochdispersen Siliciumdioxid und mindestens einem Schichtgittersilikat stammt

$X^2$    mindestens eines der Elemente der Alkalireihe

a = 3 - 10

b = 0,5 - 2

c = 3 - 10

d = 2 - 20

e = 0,01 - 2

f = Atomzahl, die sich zur stöchiometrischen Absättigung der übrigen Komponenten aus den Wertigkeiten und Anteilen ergibt.

Die Katalysatoren weisen vorzugsweise eine BET-Oberfläche von 5 bis 50 $m^2$/g, ein Gesamtporenvolumen von 0,1 bis 1 ml/g und einen mittleren Porenradius von 1 bis 15 · $10^{-8}$ m auf. Sie bewirken einen hohen Umsatz mit sehr guten Selektivitäten bei hohen Raum-Zeit-Ausbeuten und Standzeiten. Deshalb sind sie besonders gut für Einsätze im technischen Bereich geeignet. Besonders vorteilhaft sind Katalysatoren, die Antimon, Vanadium, Titan, Silicium und Kalium enthalten.

Um die erfindungsgemäßen Katalysatoren herzustellen, werden Antimon, Vanadium, Titan sowie die Elemente Silicium und die Elemente der Alkalireihe zweckmäßigerweise als Verbindungen mit Sauerstoff, in elementarer Form oder in Form von Verbindungen, die sich leicht in Sauerstoffverbindungen überführen lassen wie zum Beispiel Nitrate, Oxalate oder Carbonate, gegebenenfalls eine oder mehrere der Substanzen als Lösung oder Aufschlämmung in Wasser, eingebracht. Die so erhaltene Lösung mit Feststoffanteilen wird unter Rühren gekocht und zunächst bei einem niedrigen pH zwischen 0 und 2, vorzugsweise 0,5 und 1,5 gehalten. Gegen Ende dieser Kochphase wird das Reaktionsgemisch abgekühlt, mit einer Base, vorzugsweise Ammoniak, der pH erhöht und auf einen Wert zwischen 3 und 6, vorzugsweise 4 und 5 eingestellt und erneut gekocht. Die so gewonnene Suspension kann nach der Abkühlung auf < 50°C direkt weiterverarbeitet werden. Hierzu wird sie in einem Sprühtrockner bei Temperaturen zwischen 200 und 700°C bei Umdrehungen zwischen 20.000 und 60.000 Upm getrocknet. Abhängig von Temperatur und Drehzahl läßt sich die Korngröße gezielt einstellen. Die Abscheidung des Pulvers erfolgt an einem Zyklon. Das so erhaltene Pulver kann direkt weiterverarbeitet werden. Im Vergleich zum Stand der Technik entfällt so die aufwendige Trocknung, Mahlung und Zwischenkalzinierung.

Zur Vorbereitung der Extrusion werden dem so erhaltenen Pulver übliche Extrusionshilfsmittel und Porenbildner zugegeben, z. B. Ammoniumcarbonat, Kohlehydrate, Stärke, Cellulose oder mehrwertige Alkohole und Lösungsmittel. Durch inniges Verkneten resultiert eine pastöse Masse, die in einem Extruder, z. B. einem Schneckenextruder, durch eine Düse extrudiert wird. Dabei hängen die Länge der Extrudate von der Extrusionsgeschwindigkeit und der Durchmesser vom Extrusionswerkzeug ab. Der Zusatz an Extrusionshilfsmitteln und Porenbildnern ohne Lösungsmittel beträgt zur verarbeiteten Katalysatormenge 1 bis 30 Gew.-%, bevorzugt 5 bis 20 Gew.-%. Als Lösungsmittel eignen sich besonders gut Wasser, mit Wasser mischbare, organische Lösungsmittel, insbesondere ein- und mehrwertige Alkohole wie Methanol, Glycerin oder Glykol, oder auch Mischungen dieser Flüssigkeiten.

Die Mengenverhältnisse werden vorzugsweise so gewählt, daß in den Katalysatoren das atomare Verhältnis von Titan zu Vanadium zwischen 2 zu 1 und 8 zu 1 liegt. Die atomaren Verhältnisse von Antimon zu Vanadium liegen zweckmäßigerweise zwischen 2 zu 1 und 20 zu 1, vorzugsweise zwischen 2 zu 1 und 10 zu 1. Der Anteil an Silizium setzt sich aus hochdispersem Siliziumdioxid mit einer BET-Oberfläche von 50 bis 500 $m^2/g$, vorzugsweise von 100 bis 300 $m^2/g$ und einem thermisch vorbehandelten Schichtgittersilikat, vorzugsweise Montmorillonit mit einer BET-Oberfläche von 0,1 - 10 $m^2/g$ zusammen. Das Gewichtsverhältnis von hochdispersem Siliziumdioxid zu Schichtgittersilikat beträgt 1 zu 1 bis 1 zu 10, bevorzugt 1 zu 1 bis 1 zu 5. Der atomare Anteil von Silizium an allen Metallen beträgt 20 bis 70 %, vorzugsweise 30 bis 50 %. Das Verhältnis aller Metalle zu dem der eingesetzten Alkalimetalle liegt zwischen 20 zu 0,01 und 20 zu 5, bevorzugt zwischen 20 zu 0,01 und 20 zu 3.

In der Natur vorkommendes Schichtgittersilikat bedarf für die erfindungsgemäße Verwendung im allgemeinen einer Vorbehandlung. Es wird fein gepulvert und, zweckmäßigerweise unter ständiger Bewegung, beispielsweise in einem Drehrohr- oder Wirbelschichtofen, auf Temperaturen zwischen 900 und 1200°C erhitzt. Die Erhitzungszeit richtet sich nach der Art des Schichtgittersilikats, nach der Temperatur und nach der Art des Ofens. In den meisten Fällen wird die Substanz wenigstens eine Stunde, jedoch nicht mehr als 10 Stunden, auf Temperaturen in dem genannten Bereich gehalten. Bevorzugt wird als Schichtgittersilikat Montmorillonit und für diesen eine Behandlungszeit von 4 bis 6 Stunden bei 975 bis 1100°C.

Das hochdisperse Siliziumdioxid kann auf verschiedene Weise gewonnen worden sein, beispielsweise durch Pyrolyse von Siliziumverbindungen oder durch Ausfällung aus Lösungen von Siliziumverbindungen. Es hat zweckmäßigerweise eine BET-Oberfläche etwa von 50 bis 500 $m^2/g$, vorzugsweise von 100 bis 300 $m^2/g$.

Zur Herstellung der erfindungsgemäßen Katalysatoren werden die Ausgangssubstanzen in möglichst fein verteilter Form innig vermischt. Es hat sich als vorteilhaft erwiesen, hierbei Wasser zuzusetzen und gegebenenfalls mit Wasser mischbare organische Lösungsmittel, insbesondere ein- und mehrwertige Alkohole wie Methanol, Glycerin oder Glykol, oder auch Mischungen dieser Flüssigkeiten. Bevorzugte Arbeitsweisen für die Bereitung der Katalysatoren sind, entweder zunächst Antimon oder Antimontrioxid unter Rühren in Wasser vorzulegen und mit Salpetersäure bei Siedetemperatur zu behandeln und danach die anderen Elemente, diese als Nitrate oder Ammoniumsalze ihrer Sauerstoffsäuren beziehungsweise das Titan bevorzugt als feinteiliges Titandioxid und das Silizium als Siliziumdioxid und das Schichtgittersilikat bevorzugt als Montmorillonit, zuzugeben, oder zunächst alle Elemente in Form ihrer Oxide, Ammoniumsalze ihrer Sauerstoffsäuren oder Nitrate unter Rühren in Wasser aufzunehmen und zuletzt Salpetersäure zuzugeben. Anschließend wird der Ansatz unter Rühren und Rückfluß gekocht. Nach der Kochphase wird die Säure mit $NH_3$ unter weiterem Rühren neutralisiert und das so entstandene Produkt über einen Feststoffabscheider in den Sprühtrockner überführt. Der Sprühtrockner wird bevorzugt mit einer Strömungsgeschwindigkeit von 1 bis 2 m/sec, gemessen im Einströmrohr, einer Lufteintrittstemperatur von 400 bis 700°C bei einer Zerstäuberdrehzahl von 20.000 bis 60.000 Upm betrieben. Das so erhaltene Korn weist eine Korngröße von 1 bis 5 $\cdot$ $10^{-5}$ m auf und hat eine BET-Oberfläche von 80 bis 120 $m^2/g$.

Zur Verformung und Extrusion werden dem Katalysator Verformungs- und Extrusionshilfsmittel sowie ein Lösungsmittel oder Lösungsmittelgemisch zugesetzt und durch Kneten eine extrusionsfähige Masse erhalten. Diese Masse wird so extrudiert, daß Formkörper erhalten werden, die mindestens 3 und max. 10 mm Länge haben. Besonders geeignet sind hierfür Strangpressen, Schneckenextruder oder 2-Wellen-Extruder. Die anfallenden Formlinge werden getrocknet (Temperatur 20 bis 200°C) und nachfolgend ohne weitere Zwischenbehandlung in Gegenwart von Sauerstoff getempert.

Zur Temperung haben sich Drehrohröfen und Muffelöfen als besonders geeignet gezeigt. Eine Endtemperung zwischen 300 und 800°C, vorzugsweise zwischen 550 und 750°C, führt dabei zu den aktivsten und selektivsten Katalysatoren.

Damit wird das aufwendige Temperverfahren aus dem Stand der Technik (EP-B 0 059 414) vermieden.

Die fertigen Katalysatoren haben im allgemeinen eine BET-Oberfläche von 5 bis 50 $m^2$/g, ein Gesamtporenvolumen von 0,1 bis 1 ml/g und einen mittleren Porenradius von 1 bis 15 $\cdot$ $10^{-8}$ m. Ihre Schüttdichte ist etwa 0,8 bis 1,5 kg/l. Sie werden je nach Form und Größe in einem Festbett oder in der Wirbelschicht eingesetzt.

Die Umsetzung der Methylpyridine, insbesondere $\alpha$-, $\beta$-, $\gamma$-Methylpyridine mit Ammoniak und Sauerstoff zu den entsprechenden Cyanopyridinen erfolgt in üblicher Weise in der Gasphase. Dabei ist für die Wahl der Umsetzungsbedingungen ein breiter Spielraum gegeben. Die Umsetzung erfolgt vornehmlich ohne Anwendung von Druck oder unter geringem Überdruck bis zu etwa 3 bar bei Temperaturen zwischen 300 und 460°C, bevorzugt zwischen 320 und 440°C. Es hat sich als vorteilhaft erwiesen, den benötigten Sauerstoff als Luft zuzuführen. Je nach eingesetztem Methylpyridin ist es von Vorteil, Wasserdampf und/oder Stickstoff zuzumischen. Das Verhältnis von Methylpyridin zu Ammoniak, Sauerstoff beziehungsweise Luft und gegebenenfalls Wasserdampf und/oder Stickstoff kann in weiten Grenzen gewählt werden. Im allgemeinen ist es zweckmäßig, je Mol Methylpyridin etwa 2 bis 10 Mol, vorzugsweise 3 bis 8 Mol Ammoniak, etwa 20 bis 40 Mol, vorzugsweise 25 bis 35 Mol Luft und etwa 0 bis 20 Mol, vorzugsweise 0 bis 15 Mol Wasserdampf und/oder Stickstoff anzuwenden. Je Liter Schüttvolumen des Katalysators werden zweckmäßigerweise etwa 1 bis 2 Mol Methylpyridin pro Stunde in den Reaktor eingespeist.

In den Beispielen bedeutet % Gewichtsprozente, soweit nicht anders bezeichnet.

In den nachfolgenden Beispielen werden als Begriffe verwendet:

$$\text{Umsatz} = \frac{\text{Mole umgesetzter Kohlenwasserstoff}}{\text{Mole eingesetzter Kohlenwasserstoff}} \cdot 100 \ (\%)$$

$$\text{Ausbeute} = \frac{\text{Mole erzeugtes Produkt}}{\text{Mole eingesetzter Kohlenwasserstoff}} \cdot 100 \ (\%)$$

$$\text{R/Z-Ausb.} = \frac{\text{Masse des erzeugten Nitrils/Zeit}}{\text{Schüttvolumen des Katalysators}} \left( \frac{g}{l \cdot h} \right)$$

$$\text{Selektivität} = \frac{\text{Ausbeute}}{\text{Umsatz}} \cdot 100 \ \%$$

## Beispiel 1

2,332 kg Antimontrioxid, 469,9 g Ammoniumvanadat, 1,278 kg Titandioxid (Oberfläche 52 $m^2$/g), 1,162 kg bei 1040°C getemperter Montmorillonit mit einer BET-Oberfläche von 1 $m^2$/g und 580,5 g Siliziumdioxid mit einer BET-Oberfläche von 200 $m^2$/g (Aerosil) wurden in 12,7 kg Wasser aufgeschlämmt. Zu dieser Suspension wurden 506 g einer 10 %igen Kaliumnitratlösung unter Rühren zugegeben. Dann wurden langsam 2,14 kg 52 %ige Salpetersäure zugegeben und die Mischung auf Siedetemperatur unter Rückfluß erhitzt und 2 Stunden auf Siedetemperatur gehalten. Unmittelbar nach der Kochphase wurde mit 25 %iger wäßriger Ammoniaklösung der pH von 4,6 eingestellt und erneut für 2 Stunden unter Rückfluß gekocht. Nach dieser zweiten Kochphase wurde der Ansatz über Nacht gerührt und dann direkt zur Sprühtrocknung eingesetzt. Dazu wurde der Ansatz über einen Feststoffabscheider dem Sprühtrockner zugeführt und bei einem Luftdurchsatz von etwa 50 $m^3$/h, einer Eingangstemperatur von etwa 600°C bei einer Drehzahl von 40.000 Upm getrocknet. Das so erhaltene Pulver wurde an einem Zyklon abgeschieden und wies eine Korngröße von 2,2 bis 2,4 $\cdot$ $10^{-5}$ m mit einer BET-Oberfläche von 100 - 120 $m^2$/g auf. Das so erhaltene Katalysatorpulver wurde pro 1000 g Katalysatorpulver mit etwa 100 g Pentaerythrit innig vermischt und anschließend mit 500 g einer 10 %igen Stärkelösung zu einer pastösen Masse verknetet.

Die Masse wurde mittels einer kernprogressiven Austragsschnecke durch eine Düse mit 4 Bohrungen mit 3 mm Durchmesser extrudiert und mit einem Draht in etwa 5 mm lange Formkörper geschnitten. Die Formkörper wurden mit etwa 60°C heißer Luft vorgetrocknet und über Nacht an der Luft getrocknet. Die luftgetrockneten Formkörper können dann am nächsten Morgen zur Temperung eingesetzt werden.

Zur Temperung der Muster wurden 80 g der luftgetrockneten Formkörper in ein Temperrohr eingefüllt und 60 Min. bei der gewünschten Temperatur gehalten. Nach dem Abkühlen ist der Katalysator einsatzbereit. Seine Zusammensetzung entspricht der Formel $Si_{7,25}Ti_4V_1Sb_4K_{0,125}O_x$ (BET-Oberfläche 20 $m^2$/g, Gesamtporenvolumen 0,35 ml/g, mittlerer Porenradius 4-5 $\cdot$ $10^{-8}$ m).

**Beispiel 2**

Es wurde ein Katalyator in der gleichen Weise hergestellt, wie in Beispiel 1 beschrieben. Diesem Katalysator wurde jedoch die doppelte der im Beispiel 1 genannten Menge an Kaliumnitrat beigemischt. Das Verfahren der Sprühtrocknung, Verformung und Temperung blieb unberührt. Seine Zusammensetzung entspricht der Formel $Si_{7,25}Ti_4V_1Sb_4K_{0,25}O_x$ (BET-Oberfläche 25 m$^2$/g, Gesamtporenvolumen 0,35 ml/g, mittlerer Porenradius 4-6 $\cdot$ 10$^{-8}$ m).

**Beispiel 3**

Es wurde ein Katalysator in der gleichen Weise hergestellt, wie in Beispiel 1 beschrieben. Diesem Katalysator wurde die vierfache der im Beispiel 1 genannten Menge an Kaliumnitrat beigemischt. Das Verfahren der Sprühtrocknung, Verformung und Temperung blieb unberührt. Seine Zusammensetzung entspricht der Formel $Si_{2,75}Ti_4V_1Sb_4K_{0,5}O_x$ (BET-Oberfläche 30 m$^2$/g, Gesamtporenvolumen 0,35 ml/g, mittlerer Porenradius 5-6 $\cdot$ 10$^{-8}$ m).

**Beispiel 4**

Es wurde ein Katalysator in der gleichen Weise hergestellt, wie in Beispiel 1 beschrieben. Diesem Katalysator wurden 40 % der im Beispiel 1 genannten Menge an Kaliumnitrat beigemischt. Das Verfahren der Sprühtrocknung, Verformung und Temperung blieb unberührt. Seine Zusammensetzung entspricht der Formel $Si_{2,75}Ti_4V_1Sb_4K_{0,05}O_x$ (BET-Oberfläche 20 m$^2$/g, Gesamtporenvolumen 0,34 ml/g, mittlerer Porenradius 5-6 $\cdot$ 10$^{-8}$ m).

**Beispiel 5**

Es wurden 50 ml eines nach Beispiel 1 und bei 670°C getemperten Katalysators in ein Reaktionsrohr von 20 mm lichter Weite und 500 mm Länge eingefüllt. Stündlich wurden in das Rohr 75,4 mmol 3-Methylpyridin, 455,8 mmol Ammoniak, 2250 mmol Luft und 679 mmol Wasserdampf als Gasgemisch eingespeist. Das Reaktionsrohr wurde durch eine Salzschmelze beheizt, die auf 330, 340 und 370°C gehalten wurde. Bei jeder Temperatur wurde der Katalysator für 150 Min. mit dem Gasgemisch beaufschlagt. Beim Austritt aus dem Reaktionsrohr wurden die Gase mit Wasser gewaschen. Die Ergebnisse der katalytischen Tests befinden sich in Tabelle 1.

Tabelle 1

| Temperatur [°C] | Umsatz [%] | Ausbeute [%] | Selektivität [%] | Raum-Zeit- Ausbeute |
|---|---|---|---|---|
| 330 | 76,8 | 71,2 | 92,7 | 111,7 |
| 340 | 88,3 | 83,5 | 94,6 | 131,0 |
| 370 | 99,4 | 95,2 | 95,8 | 149,3 |

Der Umsatz bezieht sich auf die eingesetzte Menge; die Ausbeute an 3-Cyanopyridin wurde auf die eingesetzte Menge an 3-Methylpyridin bezogen. Die Raum-Zeit-Ausbeute ist in Gramm pro Liter und Stunde angegeben.

**Beispiel 6**

Es wurde wie in Beispiel 5 verfahren, jedoch wurde ein nach Beispiel 2 hergestellter Katalysator verwendet, der bei 645°C getempert wurde. Die Einspeisemengen und Temperaturen sind wie in Beispiel 5 beschrieben. Die Ergebnisse der katalytischen Tests befinden sich in Tabelle 2.

Tabelle 2

| Temperatur [°C] | Umsatz [%] | Ausbeute [%] | Selektivität [%] | Raum-Zeit- Ausbeute |
|---|---|---|---|---|
| 330 | 78,7 | 73,1 | 92,9 | 114,6 |
| 340 | 89,1 | 84,0 | 94,3 | 131,7 |
| 370 | 99,0 | 94,3 | 95,3 | 147,9 |

## Beispiel 7

Es wurde wie in Beispiel 5 verfahren, jedoch wurde ein nach Beispiel 3 hergestellter Katalysator verwendet, der bei 645°C getempert wurde. Die Einspeisemengen und Temperaturen sind wie in Beispiel 5 beschrieben. Die Ergebnisse der katalytischen Tests befinden sich in Tabelle 3.

Tabelle 3

| Temperatur [°C] | Umsatz [%] | Ausbeute [%] | Selektivität [%] | Raum-Zeit- Ausbeute |
|---|---|---|---|---|
| 330 | 69,5 | 65,7 | 94,5 | 103,0 |
| 340 | 81,7 | 77,8 | 95,2 | 122,0 |
| 370 | 97,4 | 93,0 | 95,5 | 145,9 |

## Beispiel 8

Es wurde wie in Beispiel 5 verfahren, jedoch wurde ein nach Beispiel 4 hergestellter Katalysator verwendet, der bei 700°C getempert wurde. Die Einspeisemengen und Temperaturen sind wie in Beispiel 5 beschrieben. Die Ergebnisse der katalytischen Tests befinden sich in Tabelle 4.

Tabelle 4

| Temperatur [°C] | Umsatz [%] | Ausbeute [%] | Selektivität [%] | Raum-Zeit- Ausbeute |
|---|---|---|---|---|
| 330 | 64,4 | 59,7 | 92,7 | 93,6 |
| 340 | 82,3 | 78,0 | 94,8 | 122,3 |
| 370 | 98,5 | 93,9 | 95,3 | 147,3 |

## Beispiel 9

Es wurde wie in Beispiel 5 verfahren, jedoch wurde statt 3-Methylpyridin das 2-Methylpyridin eingesetzt und statt Wasserdampf eine entsprechende Menge Stickstoff verwendet. Die Reaktionsgase wurden beim Austritt aus dem Reaktionsrohr mit N-Methylpyrrolidon gewaschen. Der Katalysator wurde, abweichend von Beispiel 5, nur bei 330 und 340°C getestet. Die Ergebnisse der katalytischen Tests befinden sich in Tabelle 5.

Tabelle 5

| Temperatur [°C] | Umsatz [%] | Ausbeute [%] | Selektivität [%] | Raum-Zeit- Ausbeute |
|---|---|---|---|---|
| 330 | 88,2 | 61,1 | 69,3 | 104,0 |
| 340 | 88,5 | 83,0 | 93,8 | 130,6 |
| 360 | 94,5 | 84,2 | 89,1 | 132,5 |

## Beispiel 10

Es wurde ein Katalysator in der gleichen Weise hergestellt, wie in Beispiel 1 beschrieben. Diesem Katalysator wurde jedoch kein Kaliumnitrat beigemischt. Das Verfahren der Sprühtrocknung, Verformung und Temperung blieb unberührt.

Es wurde wie in Beispiel 5 verfahren und ein Katalysator verwendet, der bei 711°C getempert wurde (BET-Oberfläche 20 m$^2$/g). Die Ergebnisse der katalytischen Tests befinden sich in Tabelle 6.

Tabelle 6

| Temperatur [°C] | Umsatz [%] | Ausbeute [%] | Selektivität [%] | Raum-Zeit- Ausbeute |
|---|---|---|---|---|
| 330 | 58,8 | 49,8 | 84,7 | 78,1 |
| 340 | 74,6 | 67,7 | 90,8 | 106,2 |
| 370 | 96,8 | 90,5 | 93,5 | 142,0 |

**Beispiel 11**

Eine Abschätzung des Desaktivierungsverhaltens eines mit und ohne Kalium dotierten Katalysators erfolgte dergestalt, daß der Katalysator im Labor an zwei Tagen bei jeweils unterschiedlichen Temperaturen getestet wurde. Für die Tests wurde ein nach Beispiel 1 hergestellter Katalysator verwendet, der bei 670°C getempert wurde und ein nach Beispiel 10 hergestellter, der bei 711°C getempert wurde.
Die Tests erfolgten wie in Beispiel 5 beschrieben; die Ergebnisse befinden sich in Tabelle 7, wobei die erreichten Ausbeuten verglichen werden.

Tabelle 7

| Tag | Temperatur | ohne Kalium | mit Kalium |
|---|---|---|---|
| 1 | 330 | 65,7 | 74,7 |
| 2 | 330 | 49,8 | 74,7 |
| 1 | 340 | 80,6 | 85,5 |
| 2 | 340 | 67,7 | 85,0 |
| 2 | 370 | 90,5 | 94,3 |

**Beispiel 12**

Es wurden mit den Kalium-dotierten Katalysatoren Belastungstests dahingehend durchgeführt, um eine höhere Raum-Zeit-Ausbeute zu erzielen. Dazu wurde ein nach Beispiel 1 hergestellter und bei 670°C getemperter Katalysator wie in Beispiel 5 beschrieben getestet. Abweichend von Beispiel 5 wurden jedoch stündlich 100,5 mmol 3-Methylpyridin, 200 mmol Ammoniak, 2100 mmol Luft und 450 mmol Wasserdampf als Gasgemisch eingespeist. Die Ergebnisse der katalytischen Tests befinden sich in Tabelle 8.

Tabelle 8

| Temperatur [°C] | Umsatz [%] | Ausbeute [%] | Selektivität [%] | Raum-Zeit- Ausbeute |
|---|---|---|---|---|
| 330 | 89,2 | 81,9 | 91,8 | 156,1 |

**Patentansprüche**

1.    Katalysatoren für die Umsetzung von Methylpyridinen mit Ammoniak und Sauerstoff zu den entsprechenden Cyanopyridinen, bestehend aus Verbindungen der Elemente Antimon, Vanadium, Titan mit Sauerstoff, **gekennzeichnet durch** die allgemeine Summenformel (I)

$$Sb_aV_bTi_cX^1_dX^2_eO_f$$

in der bedeuten

$X^1$     Silicium, wobei das Si aus dem bei der Herstellung eingebrachten hochdispersen Siliciumdioxid und mindestens einem Schichtgittersilikat stammt

$X^2$     mindestens eines der Elemente der Alkalireihe
          a = 3 - 10
          b = 0,5 - 2
          c = 3 - 10
          d = 2 - 20
          e = 0,01 - 2
          f = Atomzahl, die sich zur stöchiometrischen Absättigung der übrigen Komponenten aus den Wertigkeiten und Anteilen ergibt.

2.  Katalysatoren gemäß Anspruch 1,
    **dadurch gekennzeichnet,**
    daß sie eine BET-Oberfläche von 5 bis 50 m$^2$/g, ein Gesamtporenvolumen von 0,1 bis 1 ml/g und einen mittleren Porenradius von 1 bis 15 · 10$^{-8}$ m aufweisen.

3.  Katalysatoren gemäß den Ansprüchen 1 oder 2,
    **dadurch gekennzeichnet,**
    daß das atomare Verhältnis von Antimon zu Vanadium zwischen 2 zu 1 und 20 zu 1 liegt.

4.  Katalysator gemäß den Ansprüchen 1 oder 2,
    **dadurch gekennzeichnet,**
    daß das atomate Verhältnis von Titan zu Vanadium zwischen 2 zu 1 und 8 zu 1 liegt.

5.  Katalysator gemäß den Ansprüchen 1 bis 4,
    **dadurch gekennzeichnet,**
    daß der Alkalimetallanteil gemessen an der Gesamtmetallatomkonzentration der oben genannten Elemente (außer Sauerstoff) 0,01 bis 15 % beträgt.

6.  Verfahren zur Herstellung von Katalysatoren der allgemeinen Formel I,
    **dadurch gekennzeichnet,**
    daß man Mischungen aus Verbindungen der Elemente Antimon, Vanadium, Titan und Silicium mit Sauerstoff in den Formel I entsprechenden Konzentrationen, in Form einer wäßrigen Suspension und/oder Lösung bereitet, wobei sich der Anteil an Silicium durch hochdisperses Siliciumdioxid und Schichtgittersilicat eingebracht wird und das atomare Verhältnis von Vanadium zu Titan kleiner als 1 ist, das atomare Verhältnis von Antimon zu Vanadium größer als 1 ist in ihren Sauerstoffverbindungen, sowie einem oder mehreren Alkalimetallen in ihren Sauerstoffverfindungen, wobei der Alkalimetallanteil gemessen an der Gesamtmetallatomkonzentration der oben genannten Elemente (außer Sauerstoff) 0,01 bis 15 % beträgt, mit der Maßgabe, daß das Verhältnis der Alkalimetallatome zu den anderen oben genannten Metallen jeweils geringer als 1 zu 1 ist, die Suspension und/oder Lösung bei einem pH-Wert zwischen 0 und 2 unter Rückfluß umsetzt, anschließend den pH-Wert auf 3 bis 6 einstellt, erneut unter Rückfluß kocht, sprühtrocknet, das so erhaltene Pulver unter Zusatz von Extrusionshilfsmitteln, Porenbildnern und Lösungsmitteln verknetet und extrudiert und die so gewonnenen Formkörper bei Temperaturen zwischen 300 und 800°C, vorzugsweise zwischen 550 und 750°C in Gegenwart von Sauerstoff erhitzt.

7.  Verwendung der Katalysatoren nach den Ansprüchen 1 bis 5 zur katalytischen Umsetzung von Methylpyridinen mit Ammoniak und Sauerstoff zu den entsprechenden Cyanopyridinen.

8.  Verfahren zur Herstellung von Cyanopyridinen durch Umsetzung der entsprechenden Methylpyridinen,
    **dadurch gekennzeichnet,**
    daß ein Katalysator gemäß den Ansprüchen 1 bis 5 verwendet wird.

9.  Verfahren nach Anspruch 8,
    **dadurch gekennzeichnet,**
    daß je Mol Methylpyridin etwa 2 bis 10 Mol Ammoniak, etwa 20 bis 40 Mol Luft und etwa 0 bis 20 Mol Wasserdampf und/oder Stickstoff eingespeist werden und die Umsetzung bei Temperaturen zwischen 300 und 460°C erfolgt.

**Claims**

1. Catalysts for the reaction of methylpyridines with ammonia and oxygen to yield the corresponding cyanopyridines consisting of compounds of the elements antimony, vanadium, titanium with oxygen,
   characterised by
   the general empirical formula (I)

$$OSb_aV_bTi_cX_d^1X_e^2O_f$$

   in which

   $X^1$    means silicon, wherein the Si originates from the highly disperse silicon dioxide introduced during production and at least one sheet lattice silicate

   $X^2$    means at least one of the elements of the alkali metal series
   a = 3-10
   b = 0.5-2
   c = 3-10
   d = 2-20
   e = 0.01-2
   f = atomic number for stoichiometric saturation of the other components calculated from the valencies and contents.

2. Catalysts according to claim 1,
   characterised in that
   they have a BET surface area of 5 to 50 $m^2$/g, a total pore volume of 0.1 to 1 ml/g and an average pore radius of 1 to 15 · $10^{-8}$ m.

3. Catalysts according to claims 1 or 2,
   characterised in that
   the atomic ratio of antimony to vanadium is between 2:1 and 20:1.

4. Catalyst according to claims 1 or 2,
   characterised in that
   the atomic ratio of titanium to vanadium is between 2:1 and 8:1.

5. Catalyst according to claims 1 to 4,
   characterised in that
   the alkali metal content, measured relative to the total metal atom concentration of the above-stated elements (except oxygen), is 0.01 to 15%.

6. Process for the production of catalysts of the general formula I,
   characterised in that
   mixtures of compounds of the elements antimony, vanadium, titanium and silicon with oxygen in concentrations corresponding to the formula I are prepared in the form of an aqueous suspension and/or solution, wherein the silicon content is introduced by highly disperse silicon dioxide and sheet lattice silicate and the atomic ratio of vanadium to titanium is less than 1, the atomic ratio of antimony to vanadium is greater than 1 in the oxygen compounds thereof, together with one or more alkali metals in the oxygen compounds thereof, wherein the alkali metal content, measured relative to the total metal atom concentration of the above-stated elements (except oxygen), is 0.01 to 15%, with the proviso that the ratio of the alkali metal atoms to the other above-stated atoms is in each case less than 1:1, the suspension and/or solution is reacted with refluxing at a pH value of between 0 and 2, the pH value is then adjusted to 3-6, the mixture is refluxed again, spray dried, the resultant powder is kneaded with the addition of extrusion auxiliaries, pore formers and solvents and extruded and the resultant mouldings are heated at temperatures of between 300 and 800°C, preferably of between 550 and 750°C, in the presence of oxygen.

7. Use of the catalysts according to claims 1 to 5 for the catalytic reaction of methylpyridines with ammonia and oxygen to yield the corresponding cyanopyridines.

8.  Process for the production of cyanopyridines by reaction of the corresponding methylpyridines, characterised in that
    a catalyst according to claims 1 to 5 is used.

9.  Process according to claim 8,
    characterised in that,
    for each mol. of methylpyridine, approx. 2 to 10 mol. of ammonia, approx. 20 to 40 mol. of air and approx. 0 to 20 mol. of steam and/or nitrogen are introduced and the reaction proceeds at temperatures of between 300 and 460°C.

**Revendications**

1.  Catalyseurs pour la transformation de méthylpyridines avec de l'ammoniac et de l'oxygène en cyanopyridines correspondantes, constitués par des composés des éléments antimoine, vanadium, titane avec de l'oxygène, caractérisés par la formule brute générale (I)

    $$Sb_aV_bTi_cX_d^1X_e^2O_f$$

    dans laquelle

    $X^1$   signifie du silicium, le Si provenant du dioxyde de silicium à forte dispersion et d'au moins un silicate à réseau en couche introduits lors de la préparation
    $X^2$   signifie au moins un des éléments de la série des alcalins,
        a = 3 - 10
        b = 0,5 - 2
        c = 3 - 10
        d = 2 - 20
        e = 0,01 - 2
        f = nombre atomique obtenu à partir des valences et des proportions pour la saturation stoechiométrique des autres composants.

2.  Catalyseurs selon la revendication 1, caractérisés en ce qu'ils présentent une surface BET de 5 à 50 $m^2$/g, un volume total de pores de 0,1 à 1 ml/g et un rayon moyen des pores de 1 à 15.$10^{-8}$m.

3.  Catalyseurs selon les revendications 1 ou 2, caractérisés en ce que le rapport atomique entre l'antimoine et le vanadium est compris entre 2:1 et 20:1.

4.  Catalyseur selon les revendications 1 ou 2, caractérisé en ce que le rapport atomique entre le titane et le vanadium est compris entre 2:1 et 8:1.

5.  Catalyseur selon les revendications 1 à 4, caractérisé en ce que la proportion de metal alcalin, mesurée par rapport à la concentration totale en atomes métalliques des éléments susmentionnés (à l'exception de l'oxygène), est comprise entre 0,01 et 15 %.

6.  Procédé pour la préparation de catalyseurs de formule générale I, caractérisé en ce qu'on prépare des mélanges de composés des éléments antimoine, vanadium, titane et silicium avec de l'oxygène en des concentrations qui correspondent à la formule I, sous forme d'une suspension et/ou solution aqueuses, la proportion en silicium étant introduite sous forme de dioxyde de silicium à forte dispersion et de silicate à réseau en couche et le rapport atomique entre le vanadium et le titane étant inférieur à 1, le rapport atomique entre l'antimoine et le vanadium étant supérieur à 1 dans leurs composés oxygénés, et présentant un ou plusieurs métaux alcalins dans leurs composés oxygénés, la proportion de métaux alcalins mesurée par rapport à la concentration totale en atomes métalliques des éléments susmentionnés (à l'exception de l'oxygène) étant comprise entre 0,01 et 15 %, sous réserve que le rapport entre les atomes de métaux alcalins et les autres métaux susmentionnés soit chaque fois inférieur à 1:1, en ce qu'on transforme la suspension et/ou la solution à un pH compris entre 0 et 2 sous reflux, en ce qu'on règle ensuite le pH entre 3 et 6, en ce qu'on amène à nouveau à ébullition sous reflux, en ce qu'on sèche par pulvérisation, en ce qu'on pétrit et extrude la poudre ainsi obtenue en ajoutant des adjuvants d'extrusion, des agents porogènes et des solvants et en ce qu'on chauffe les corps moulés ainsi fabriqués à des températures comprises entre 300 et 800 °C, de préférence entre 550 et 750 °C, en présence d'oxygène.

7. Utilisation des catalyseurs selon les revendications 1 à 5 pour la transformation catalytique de méthylpyridines avec de l'ammoniac et de l'oxygène en cyanopyridines correspondantes.

8. Procédé pour la préparation de cyanopyridines par transformation des méthylpyridines correspondantes, caractérisé en ce qu'on utilise un catalyseur selon les revendications 1 à 5.

9. Procédé selon la revendication 8, caractérisé en ce qu'on alimente, par mole de méthylpyridine, environ 2 à 10 modes d'ammoniac, environ 20 à 40 modes d'air et environ 0 à 20 modes de vapeur d'eau et/ou d'azote et en ce que la réaction se déroule à des températures comprises entre 300 et 460 °C.